# EUROPEAN PATENT SPECIFICATION

(11) **EP 0 814 801 B1**
(45) Date of publication and mention of the grant of the patent: **30.07.2003**
(21) Application number: 96905942.7
(22) Date of filing: 11.03.1996
(51) Int. Cl.: A61K 31/22, A61K 31/00, A61K 31/19, A61P 9/10

(54) **USE OF A PYRUVATE DEHYDROGENASE ACTIVATOR FOR THE TREATMENT OF ISCHAEMIA IN LIMBS**
VERWENDUNG EINES PYRUVATDEHYDROGENASEAKTIVATORS ZUR BEHANDLUNG DER ISCHÄMIE IN GLIEDERN
UTILISATION D'UN ACTIVATEUR DE LA PYRUVATE DEHYDROGENASE POUR LE TRAITEMENT DE L'ISCHEMIE DANS LES MEMBRES

(30) Priority: 14.03.1995 GB 9505082
(43) Date of publication of application: 07.01.1998
(73) Proprietor: AstraZeneca AB, 151 85 Södertälje (SE)
(72) Inventor: CONSTANTIN-TEODOSIU, Dumitru, University Park Nottingham NG7 2RD (GB); TIMMONS, James, Archibald, University Park Nottingham NG7 2RD (GB); GREENHAFF, Paul, Leonard, University Park Nottingham NG7 2RD (GB); POUCHER, Simon, Martin, Cheshire SK10 4TG (GB)
(74) Representative: Bill, Kevin
(86) International application number: GB9600555
(87) International publication number: WO96028151

(56) References cited:
- AM. REV. RESPIR. DIS., vol. 145, 1992, pages 345-54, XP000611607 CURTIS: "Regional and Systemic Oxygen delivery/Uptake Relations and Lactate Flux in Hyperdynamic, Endotoxin-treated Dogs."
- J. APPL. PHYSIOL., vol. 74, no. 4, 1993, pages 1712-8, XP000611603 CONSTANTIN-TEODOSIU, D. ET AL.: "PDC activity and acetyl group accumulation in skeletal muscle during isometric contraction"
- J. APPL. PHYSIOL., vol. 66, 1989, pages 591-597, XP000609079 CARRARO, F.: "Effect of dichloroacetate on lactate concentration in exercising humans"
- AM. J. PHYSIOL. (AJPHAP,00029513);90; VOL.259 (4, PT. 2); PP.H1079-H1085, UNIV. ALBERTA;FAC. MED.; EDMONTON; T6G 2S2; AB; CAN. (CA), XP002018148 MCVEIGH J J ET AL: "Dichloroacetate stimulation of glucose oxidation improves recovery of ischemic rat hearts"
- JPN. HEART J. (JHEJAR,00214868);87; VOL.28 (4); PP.531-7, TOKUSHIMA UNIV.;SCH. MED.; TOKUSHIMA; 770; JAPAN (JP), XP000608956 MATSUOKA S ET AL: "Effects of dichloroacetate on the mechanical function of the isolated ischemic heart"
- ACTA MED. SCAND., SUPPL. (AMSSAQ);76; VOL.587,; PP.29-34, UNIV. TROMSOE;INST. MED. BIOL.; TROMSOE; NORWAY, XP000608959 MJOES O D: "Effect of reduction of myocardial free fatty acid metabolism relative to that of glucose on the ischemic injury during experimental coronary artery occlusion in dogs"
- FARMACO, ED. PRAT. (FRPPAO);71; VOL.26 (9); PP.544-56, UNIV. PAVIA;IST. GERONTOL. GERIATR.; PAVIA; ITALY, XP002018149 GIAROLA P ET AL: "Antithrombic and antidyslipemic effects of diisopropylammonium dichloroacetate in animals and humans"
- GAZZ. MED. ITAL., vol. 129, no. 11, 1970, pages 464-471, XP000609088 MOSELLA, G. ET AL.: "Trattamento locale di ulcere sperimentali nel coniglio(prodotte in animali normali sottoposti ad ischemia e stasi venosa locale da insufficienza arteriosa, venosa e fleboarteriosa) con un pomata contente DED e sostanze capillaro-protettrici."
- BIOCHEM. JOURNAL, vol. 219, 1984, pages 635-646, XP000611687 FULLER S.J. ET AL.: "Reversible phosphorylation of pyruvate dehydrogenase in rat skeletal-muscle mitochondria."
- DRUG DEVELOPMENT RESEARCH, vol. 35, 1995, pages 130-136, XP000611456 ESPINAL, J. ET AL.: "Inhibition of Pyruvate dehydrogenase Kinase by Halogenated Acetophenones"

## Description

This invention relates to the preparation of a medicament for the treatment of ischaemia in limbs and in particular to the treatment of intermittent claudication.

The increase in the size of the elderly population, together with factors such as a high incidence of cigarette smoking and poor diet, have led to an increase in the number of patients showing signs of peripheral arterial disease.

The clinical manifestations of ischaemia of a limb include, for example, muscle pain experienced on exercise but relieved by rest. This phenomenon is generally known as intermittent claudication. Typically, the patient experiences a cramp-like pain in a leg after walking a relatively short distance of. for example. 100m. On resting for a few minutes, the pain usually disappears but reappears again after walking the same short distance.

During glycolysis glucose is converted to pyruvate. The degradation of amino acids such as alanine, serine and cysteine also generates pyruvate. Pyruvate has a number of metabolic fates depending on the nature of the tissue. One reaction of pyruvate which takes place in the mitochrondrial compartment of cells is its oxidative decarboxylation to acetyl CoA. This reaction is catalysed by the enzyme complex pyruvate dehydrogenase (PDH).

Dichloroacetate (DCA) is widely reported in the literature as. inter alia, having the property of inhibiting PDH kinase. Animal studies with PDH kinase inhibitors reported by Hatta & Atomi (1994. Biochemistry of Exercise Meeting. Aberdeen. July. 1994) showed that DCA increased the oxidation of lactate and increased treadmill exercise duration of normal mice by 20%. It has also been reported (Ludvik et al. 1993, Pflüg. Arch 423:251-254) that DCA reduces lactate production in volunteers during maximal and 50% maximal workload by up to 30% and increased maximal work capacity by 10%. In angina patients undergoing catheterisation. DCA increased stroke volume by 13% in the absence of changes in heart rate or left ventricular inotropic state (LV dp/dtmax.). Myocardial efficiency is also increased from 24% to 32% (Wargovich et al 1988. Am J Cardiol 61:65-70). Beneficial effects of DCA in patients with class III heart failure has also been reported (Chatergee et al. 1994; J Am Coil Cardiol. 23: 1617-1624).

The present invention is based on the discovery that activation of pyruvate dehydrogenase gives rise to an improvement in ischaemic muscle function in limbs and hence in medical conditions such as intermittent claudication. In particular, the present invention is based on the unexpected discovery that an improvement in ischaemic muscle function in limbs and hence in medical conditions such as intermittent claudication may be obtained by inhibiting PDH kinase.

According ta the present invention there is provided the use of an agent which activates pyruvate dehydrogenase to prepare a medicament which activates pyruvate dehydrogenase in warm blooded animals such as man and is for the treatment of ischaemia in limbs.

In particular, the medicament may be used to treat intermittent claudication.

The medicament is for use in a method of treating ischaemia in limbs in warm blooded animals such as man, comprising administering an effective amount of an agent which activates pyruvate dehydrogenase.

In particular there is provided a method of treating intermittent claudication in warm blooded animals such as man, comprising administering an effective amount of an agent which activates pyruvate dehydrogenase.

Thus, the agents used according to the present invention are useful in treating peripheral vascular disease in a method of treating peripheral vascular disease in warm blooded animals such as man comprising administering an effective amount of an agent as herein defined.

The major sources of energy substrate for skeletal muscle are fatty acids and carbohydrate, with respiratory quotients (RQ) of 0.7 and 1.0 respectively. Under resting conditions skeletal muscle normally has a RQ close to 0.7 which increases to around 1.0 during strenuous exercise. In claudicants however, the degree to which substrate supply is switched is impaired since the respiratory quotient increase is limited during exercise of the symptomatic limb (Lundgren et al. 1988. Am J Physiol 255. H1156 - H1164). The reduced RQ is indicative of a higher dependence upon fatty acids for ATP synthesis in the ischaemic limb. Although the symptomatic limb adapts to low flow by increasing oxygen extraction this is not sufficient to sustain fatty acid oxidation as a means of ATP production which requires 14% more oxygen to produce ATP than required for carbohydrate sources.

We believed that if the metabolism within the symptomatic limb could be switched to the more oxygen efficient carbohydrate metabolism the ability to maintain ATP levels. and hence muscle function, could be improved.

One approach we have investigated to increase the utilisation of carbohydrate is to increase the conversion of pyruvate to acetyl-CoA by activation of pyruvate dehydrogenase (PDH). Activity of PDH is regulated by a number of factors, the most important of which is the degree of phosphorylation of the E1 sub-unit. Phosphorylation results in inactivation of the PDH complex and this process is regulated by a phosphatase and a kinase enzyme. We believed that inhibition of PDH kinase would inhibit the phosphorylation of PDH and give rise to increased pyruvate and hence carbohydrate oxidation.

We unexpectedly found that if, prior to exercise, the more oxygen efficient carbohydrate metabolism is activated, then a pool of acetyl groups is produced. This pool is available for entry into the TCA cycle during exercise resulting in oxygen efficient ATP production and improvement in skeletal muscule function.

Dichloroacetate (DCA), that is dichloroacetic acid and salts thereof, is an agent known to inhibit PDH kinase.

The agent may comprise any agent which activates PDH. This property may be determined using standard laboratory techniques well known to those skilled in the art. For example. agents capable of activiating PDH may be identified by measuring PDH activity using methods based on the rate of NADH formation. ¹⁴CO₂ formation or acetyl-CoA formation. A particularly suitable method is described by Dumitru Constantin-Teodosiu. Doctoral Thesis "Regulation of pyruvate dehydrgenase complex activity and acetyl group formation in skeletal muscle durinmg exercise", Stockholm 1992, available from the Department of Clinical Chemistry, Karolinska Institute, Huddinge University Hospital, S-141 86 Huddinge Sweden. This method is also reported by Constantin-Teodosiu D et al.. "A sensitive radioisotopic assay of pyruvate dehydrogenase complex in human muscle tissue". Anal. Biochem.. Volume 198. p347-351, 1991.

Particularly suitable assays for identifying compounds which activate PDH include, for example, the methods described by Kerby and Randle, Biochem Journal, 231, (1985), 523-529 or Brooks and Storey, Analytical Biochemistry, 212, (1993), 452-456.

In general, it is preferred that the agent comprises an agent which inhibits the enzyme pyruvate dehydrogenase kinase (PDH kinase) and hence according to a particular embodiment of the present invention there is provided the use of an agent which activates pyruvate dehydrogenase to prepare a medicament for the treatment of ischaemia in limbs (and in particular for the treatment of intermittent claudication).

Thus preferred agents will comprise chemical compounds which inhibit PDH kinase. This property may be determined using standard laboratory techniques well known to those skilled in the art, for example, methods based on those mentioned above in relation to identification of PDH activators, such as those described by Kerby and Randle, Biochem Journal, 231, (1985), 523-529 or Brooks and Storey, Analytical Biochemistry, 212, (1993), 452-456. The modification of the assays mentioned above in relation to PDH to assays to identify PDH kinase inhibitors is routine modification to those skilled in the art.

As mentioned above, DCA is agent known to inhibit PDH kinase. The experimental methods described below use DCA to demonstrate that PDH kinase inhibitors can reduce the rate of fatigue in contracting skeletal muscle of anaesthetised dogs which has blood flow limited to the same extent as patients with intermittent claudication. Thus, the experimental methods described below demonstrate that agents which are able to activate PDH, and in particular agents which are able to inhibit PDH kinase are useful in treating intermittent claudication.

Preferred agents which inhibit PDH kinase include, for example, Dichloroacetic acid, derivatives thereof and salts. Derivatives will include, for example, esters derivatives. Suitable esters will include, for example, alkyl, cycloalkyl, cycloalkylalkyl and phenylalkyl esters in which the phenyl moiety may be optionally substituted.

In a particular embodiment of the present invention there is provided the use of a compound of formula I, or a pharmaceutically acceptable salt thereof. 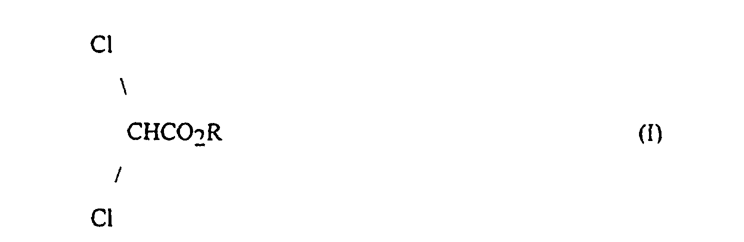 wherein:
R is selected from hydrogen, (1-10C)alkyl, (3-10C)cycloalkyl, (3-10C)cycloalkyl(1-10C)alkyl and phenyl( 1-10C)alkyl in which the phenyl moiety may optionally bear one or more substituents.
   to prepare a medicament for the treatment of ischaemia in limbs, and in particular, to prepare a medicament for the treatment of intermittent claudication.

Examples of values for optional substituents for a phenyl moiety include, for example, values independently selected from halogeno, (1-4C)alkyl, (3-6C)alkenyl, (1-4C)alkoxy, cyano, trifluoromethyl, nitro, carboxy, amino, (1-4C)alkylamino, dialkylamino of up to six carbon atoms, (1-4C)alkylthio, (1-4C)alkylsulphinyl, (1-4C)alkylsulphonyl and (1-4C)alkylenedioxy.

Particular values for optional substituents which may be present on a phenyl moiety include. by way of example:
for halogeno, fluoro, chloro and bromo:
for alkyl, methyl, ethyl and propyl;
for alkenyl, allyl and 2-methyl-2-propenyl;
for alkoxy, methoxy, ethoxy and propoxy:
for alkylamino, methylamino and ethylamino:
for dialkylamino, dimethylamino and diethylamino:
for alkylthio, methylthio and ethylthio:
for alkylsulphinyl, methylsulphinyl and ethylsulphinyl;
for alkylsulphonyl, methylsulphonyl and ethylsulphonyl: and
for alkylenedioxy, methylenedioxy and isopropylidenedioxy.

Particular values for R include, by way of example:

| | |
|---|---|
| for alkyl | (1-6C)alkyl, such as, methyl, ethyl, propyl, sopropyl, butyl, pentyl or hexyl: |
| for phenylalkyl | phenyl( 1-4C)alkyl, such as, benzyl, phenylethyl or phenylpropyl; |
| for cycloalkyl | 3-6C)cycloalkyl, such as, cyclopropyl, cyclobutyl, cyclopentyl or cyclohexyl; |
| for cycloalkylalkyl | (3-6C)cycloalkyl(1-4C)alkyl, such as, cyclopropylmethyl, cylopentylmethyl, cyclohexylmethyl or 2-(cyclohexyl)ethyl. |

In one embodiment of the present invention, the compound is of formula I, or a pharmaceutically acceptable salt thereof, in which R is hydrogen.

In a further embodiment of the present invention, the compound is of formula I in which R is selected from (1-10C)alkyl. (3-10C)cycloalkyl, (3-10C)cycloalkyl(1-10C)alkyl and phenyl(1-10C)alkyl in which the phenyl moiety may optionally bear one or more substituents. Particular, preferred and specific values include the values mentioned hereinbefore.

A suitable pharmaceutically-acceptable salt of the present invention comprises those formed with a base which affords a pharmaceutically acceptable cation. Suitable bases include an alkali metal salt (such as a sodium or potassium salt), an alkaline earth metal salt (such as a calcium or magnesium salt), an ammonium salt or a salt with an organic base which affords a physiologically-acceptable cation such as a salt with methylamine, dimethylamine, triethylamine, piperidine or morpholine.

The compounds, used according to the present invention may be obtained by standard procedures of organic chemistry already known to be applicable to the preparation of structurally analogous compounds. Such procedures for the preparation of the compounds of formula I, or pharmaceutically acceptable salts thereof, are illustrated by the following preferred processes in which R may have any of the meanings defined hereinbefore.

Thus, compounds of formula I in which R is other than hydrogen may be prepared by reacting the compound of formula I (Cl₂CHCO₂R) in which R is hydrogen (that is dichloroacetic acid) with a compound of formula ROH under standard conditions used for esterifying carboxylic acids. Thus. the reaction will. in general , be carried out in the presence of an acid catalyst and. usually, in the presence of a suitable solvent. It will be appreciated that the alcohol (ROH) itself may be used as solvent. Suitable catalysts include mineral acids such as concentrated sulphuric acid or hydrochloric acid and organic acids such as p-toluenesulphonic acid. Suitable solvents include inert hydrocarbons such as toluene. The reaction may be facilitated by using an excess of the alcohol (ROH) and/or removing water generated during the course of the reaction by azeotropic distillation or by use of molecular sieves.

The compounds of formula I in which R is other than hydrogen may also be prepared by reaction of dichloroacetic acid with the corresponding alcohol of formula ROH in the presence of a dehydrating agent. Suitable dehydrating agents include. for example, dicyclohexylcarbodiimide, N.N'-carbonyldiimidazole and diethyl azodicarboxylate/triphenylphosphine. The reaction may be carried out in an inert solvent such as a hydrocarbon solvent (for example toluene) at a temperature from ambient to the reflex temperature of the reaction mixture.

Dichloroacetic acid and salts thereof are generally available and are commercially available.

When a pharmaceutically-acceptable salt of a compound of the formula I is required, it may be obtained, for example, by reaction of said compound with the appropriate base (which affords a physiologically acceptable cation), or by any other conventional salt formation procedure. In some cases the compound may near a basic group or moiety such that salts may also be prepared by reaction of the compound with the appropriate acid (which affords a physiologically acceptable anion).

As mentioned above, the compounds may be used to treat of ischaemia in limbs and in particular, they may be used to treat intermittent claudication. When used to treat diseases and medical conditions such as intermittent claudication it is envisaged that a compound of formula I (or a pharmaceutically acceptable salt thereof) will be administered orally, intravenously, or by some other medically acceptable route so that a dose in the general range of, for example, 0.01 to 500 mg per kg body weight is received. An example of dose envisages is one in the genaral range of 35-50 mg per kg body weight. However it will be understood that the precise dose administered will necessarily vary according to the nature and severity of the disease, the age and sex of the patient being treated and the route of administration.

In general, the compounds of formula I (or a pharmaceutically-acceptable salt thereof) will usually be administered in the form of a pharmaceutical composition, that is together with a pharmaceutically acceptable diluent or carrier, and such a composition is provided as a further feature of the present invention. The compound will be present in an amount effective to treat the disease to be treated, for example ischaemia in limbs.

A pharmaceutical composition prepared according to the present invention may be in a variety of dosage forms. For example, it may be in the form of tablets, capsules, solutions or suspensions for oral administration, in the form of a suppository for rectal administration; in the form of a sterile solution or suspension for parenteral administration such as by intravenous or intramuscular injection.

A composition may be obtained by conventional procedures using pharmaceutically acceptable diluents and carriers well known in the art. Tablets and capsules for oral administration may conveniently be formed with a coating, such as an enteric coating (for example, one based on cellulose acetate phthalate), to minimise dissolution of the active ingredient of formula I (or a pharmaceutically-acceptable salt thereof) in the stomach or to mask unpleasant taste.

The invention will now be further described by reference to the accompanying, non-limiting, experimental methods. In the following expermimental methods we have used the PDH kinase inhibitor dichloroacetate to determine if inhibitors of PDH kinase can reduce the rate of fatigue in contracting skeletal muscle of anaesthetised dogs which has blood flow limited to the same extent as patients with intermittent claudication. Suitable doses of DCA include these in the range 30 to 300 mg.kg⁻¹. In the experimental methods which follow DCA was administered (at a dose of 300mg.kg⁻¹) as the sodium salt of dichloroacetic acid.

### Experimental methods

Female Alderley Park Beagle dogs (12-18 kg) were pre-medicated with morphine (10 mg, i.m.) 30 minutes prior to induction of anaesthesia with sodium pentobarbitone (45 mg kg-1, i.v., Sagatal.). Each dog was intubated with an endotracheal tube and ventilated artificially with room air using a positive pressure respiration pump (model 16/24, Palmer Bioscience). Ventilation was initiated with room air at a rate of 17 strokes per min and stroke volume of 250 ml. Rectal temperature was constantly monitored and maintained (range 36.2°C - 38.6°C) with a homeothermic heating blanket (Harvard Instruments, Edenbridge, Kent. UK).

### Surgery

The left external jugular vein was cannulated for continuous administration of anaesthetic (sodium pentobarbitone 12 mg/ml at a rate of 5 - 6 ml/hour) using an infusion pump (injectomat S. Fresenius. Villiers, France). The right brachial artery was cannulated and connected to a pressure transducer. All pressures were measured using strain gauge manometers ( PDCR 75. Druck Ltd. Barendeecht, Netherlands) attached to d.c. bridge amplifiers (Lectromed, MT8P. St. Peter, Jersey). Pressure transducers were calibrated at the start of each experiment using a column of mercury. Pulse rate was electronically derived from this pressure signal. The left brachial artery was cannulated for collection of arterial blood samples and used to monitor the blood gas status of the animals (280 Blood Gas System. Ciba-Corning, Medfield, M.A., USA). The right antecubital vein was cannulated for infusion of heparin (1 iu kg-1 min-1, Multiparin) given 30 minutes before connection of the extracorporeal perfusion circuit. The left antecubital vein was cannulated for administration of 1M HCO3-(Polyfusor, Fresenius Healthcare, Basingstoke, U.K.) to maintain arterial pH within the normal range.

The gracilis muscle of both limbs was exposed using diathermy (V.I.C.3, The Genito-Urinary Mfg Co Ltd. U.K.) and blunt dissection techniques. The gracilis muscles of both hind limbs were exposed and vascularly isolated leaving only arterial supply and venous return of the gracilis muscle. To achieve this all branches of the femoral artery and vein between the deep femoral and popliteal arteries and veins, with the exception of branches supplying the gracilis muscle were ligated. The mid caudal femoral vein was cannulated and the cannula advanced into the femoral vein so that its tip was located distal to the gracilis vein. The popliteal vein was tied off distal to the medial saphenous vein to prevent venous return from the lower and deep part of the limb. All other branches that arose from the femoral or saphenous vein and not arising from the gracilis muscle were tied. The popliteal artery in both legs was cannulated and attached to a pressure transducer for recording gracilis muscle perfusion pressures.

The obturator nerves were identified and a small section dissected free and crushed centrally. Stimulating electrodes were placed the distal portion of each nerve. Muscle contraction was initiated using stimulating parameters of 0.1 msec duration. 10V. 0.3 - 3 Hz. The muscles were dissected free at their origin and insertion. Strong threads (75% polyester/25%cotton, were attached to the tendons of origin of the muscle were attached to a secure post which served as an anchor. Threads attached to the tendons of insertion were attached to force transducers (FTC10, Grass, Quincy, M.A., USA) for recording isometric tension. Muscle fatigue was measured by the equation: fatigue = (peak tension - end tension) / peak tension. Blood flow to the left gracilis muscle was allowed to change during muscle contraction with blood flow measured with an electromagnetic flow probe (10 - 14 mm circumference. Carolina Medical Electronics. King, North Carolina, U.S.A.) placed around the femoral artery. Zero blood flow was determined by means of a snare occluder placed distal to the flow probe. In situ calibration of the flow probe was completed at the end of the experiment using the dogs own blood. The blood flow to the right gracilis muscle was fixed at a constant rate. Following 30 minutes of heparin infusion (1 iu kg- 1 min-1 i.v.) the right femoral artery was cannulated proximally and distally and attached to a roller perfusion pump (minipuls3, Gilson, Villiers le bel, France). The right gracilis muscle was pump perfused at a constant flow rate sufficient to match arterial blood pressure. An equilibration period of approximately, 30 minutes, during which time blood gases were measured and corrected if necessary, was allowed before commencement of experimental protocol. All parameters were recorded on an 8 channel chart recorder (Graftech Linearcorder. Mk 8 WR3500. Nantwich, U.K.

It was found that muscle fatigue was reduced from 46.5% (±3.6%) to 25.0 % (±3.2%) folowing treatment with DCA.

### Protocol

Animals were divided into DCA treated and vehicle (isotonic saline) treated groups. DCA or vehicle was given after completion of the surgery by a constant infusion, 45 minutes later the contraction was started. Muscle fatigue was measured in both normal and flow limited muscles. Arterial blood samples were taken from the brachial artery and venous blood samples were taken from each gracilis muscle at rest, during contraction and following recovery. The contraction period was 20 minutes of stimulation of 3 Hz. Blood samples (0.4 ml) being taken for blood gas analysis and analysed immediately. Parameters recorded were pH, pCO2. pO2. oxygen saturation and total Haemoglobin. Further samples (1.0 ml) were taken for analysis of lactate, glucose and non esterified fatty acid (NEFA) concentration.

### Analytical methods.

These samples were taken into 0.05 ml 3.2% trisodium citrate and centrifuged immediately (centrifuge 5415, Eppendorf, 13,000 rpm for 5 mins). Plasma was removed immediately and aliquoted for each assay. Analysis of lactate (reagent lactate kit. Sigma Diagnostics) was carried out promptly. Plasma samples for glucose analysis were kept on ice and measured at the end of the experiment (Glucose autostat GA1120. Clandon). Plasma samples for NEFA were frozen for later analysis (NEFA C kit, ACS-ACOD method, Wako). Recovery samples were taken when blood pressure and blood flow measurements were returned to basal values, usually within 15 minutes following termination of stimulation.

### Muscle metabolites

Following resting blood samples and after drug/vehicle infusion a resting muscle biopsy (6mm diameter Bergstrom needle, Stille, Sweden and 6mm diameter Allendale needle, Edinburgh, Scotland), was taken from each muscle. After 20 minutes of stimulation the muscle was then freeze clamped, while still being stimulated, and a thin portion of muscle was excised and frozen in liquid nitrogen (The whole process took less than 6 seconds with the top layer of muscle being frozen within 1 second). The muscle samples were then stored in liquid nitrogen before a portion was freeze dried and analysed.

The muscle sample was divided into two portions, one for analysis of ATP, phosphocreatine (PCr), creatine (Cr) and intramuscular lactate (Harris, R et al., Scand. Journal Clin Lab Invest., 33, 109-120, 1974). The other portion of muscle was stored wet in liquid nitrogen. Briefly, the first portion of muscle was freeze dried, dissected free of visible connective tissue and blood and then powdered and was extracted in ice cold perchloric acid (0.5M) and neutralised with potassium bicarbonate (2.2M). Glycogen content was assayed using the method described by Harris et al. 1974 (see above).

Acetylcarnitine. free carnitine were assessed, in the freeze dried extract, by the method described by Cederblad et al.. Anal. Biochem., 185, (1990), 274-278. PDH activity (rest & exercise, active and total) from a sample of wet tissue using the method of Constantin-Teodosiu D et al., "A sensitive radioisotopic assay of pyruvate dehydrogenase complex in human muscle tissue". Anal. Biochem., Volume 198. p347-351, 1991. This method is also described by Dumitru Constantin-Teodosiu, Doctoral Thesis "Regulation of pyruvate dehydrgenase complex activity and acetyl group formation in skeletal muscle durinmg exercise". Stockholm 1992, from the Department of Clinical Chemistry, Karolinska Institute, Huddinge University Hospital, S-141 86 Huddinge Sweden.

| | PCr | Lactate | Ac.carn. | Free carnitine |
|---|---|---|---|---|
| **Control** | | | | |
| Resting | 58.3±3.4 | 10.4±1.4 | 1.9±0.3 | 20.9±1.3 |
| Normal flow | 45.0±5.2 | 11.5±3.3 | 9.1±1.8 | 17.7±1.6 |
| Resricted flow | 28.5 ±6.9 | 53.7 ±12 | 12.5 ±1.7 | 13.7±1.8 |

| **DCA** | | | | |
|---|---|---|---|---|
| Resting | 55.1±2.5 | 5.7±1.4 | 19.6±1.0 | 5.4±1.6 |
| Normal flow | 44.7±3.2 | 5.4±1.6 | 9.6±3.2 | 15.5±2.5 |
| Resricted flow | 38.9 ±3.0 | 18.1±4.0 | 16.9±2.6 | 9.6±2.2 |
| PCr = phosphocreatine | | | | |
| Ac.carn. = acetyl carnitine | | | | |

Changes in Pcr and lactate were reduced with DCA. Acetyl group availabilirty was higher both at rest and during contraction (RF muscle) in the DCA treated group. Fully transforming PDC allows for agreater flux of pyruvate through PDC and hence reduces the anaerobic contribution to ATP regeneration and minimises lactate accumulation.

In particular, the studies described above demonstrated that increasing acetyl group availability at rest resulted in a greater oxidative contribution to ATP regeneration, a substantial reduction in anaerobic metabolism, and a marked improvement in skeletal muscle contractile function during ischemic conditions. Therefore, we believe (though we do not wish to be bound to this theory) that DCA's principal action may be to reduce lactic acid accumulation and the associated deleterious effects of anaerobic metabolism.

This work is discussed by Timmons. J.A et al. J. Clin. Invest., volume 97, Number 3, February 1996. 879-883 and is incorporated herein by reference.

Illustrative pharmaceutical dosage forms suitable for presenting the compounds of the invention for therapeutic or prophylactic use include the following tablet and capsule formulations, which may be obtained by conventional procedures well known in the art of pharmacy and are suitable for therapeutic or prophylactic use in humans:-

| (a) **Tablet I** | **mg/tablet** |
|---|---|
| Compound Z* | 1.0 |
| Lactose Ph. Eur. | 93.25 |
| Croscarmellose sodium | 4.0 |
| Maize starch paste (5% w/v aqueous paste) | 0.75 |
| Magnesium stearate | 1.0 |

| (b) **Tablet II** | **mg/tablet** |
|---|---|
| Compound Z* | 50 |
| Lactose Ph. Eur | 223.75 |
| Croscarmellose sodium | 6.0 |
| Maize starch | 15.0 |
| Polyvinylpyrrolidone (5% w/v aqueous paste) | 2.25 |
| Magnesium stearate | 3.0 |

| **(c) Tablet III** | **mg/tablet** |
|---|---|
| Compound Z* | 100 |
| Lactose Ph. Eur. | 182.75 |
| Croscarmellose sodium | 12.0 |
| Maize starch paste (5% w/v aqueous paste)2.25 | |
| Magnesium stearate | 3.0 |

| **(d) Capsule** | **mg/capsule** |
|---|---|
| Compound Z* | 10 |
| Lactose Ph.Eur. | 488.5 |
| Magnesium stearate | 1.5 |

| | |
|---|---|
| Note * The active ingredient Compound Z is a compound of formula I, or a salt thereof. for example DCA. | |

The tablet compositions (a) - (c) may be enteric coated by conventional means , for example, with cellulose acetate phthalate.

## Claims

1. The use of an agent which activates pyruvate dehydrogenase to prepare a medicament which activiates pyruvate dehydrogenase in warm blooded animals such as man for the treatment of ischaemia in limbs.

2. The use as claimed in claim 1 wherein the medicament is for the treatment of intermittent claudication.

3. The use as claimed in claim 1 or 2 wherein the agent comprises an agent which inhibits PDH kinase.

4. The use as claimed in any one of claims 1 to 4 wherein the agent comprises dichloroacetic acid, a derivative thereof, or a salt thereof.

5. The use as claimed in claim 1, 2, 3 or 4 wherein the agent comprises a compound of formula I, or a pharmaceutically acceptable salt thereof. 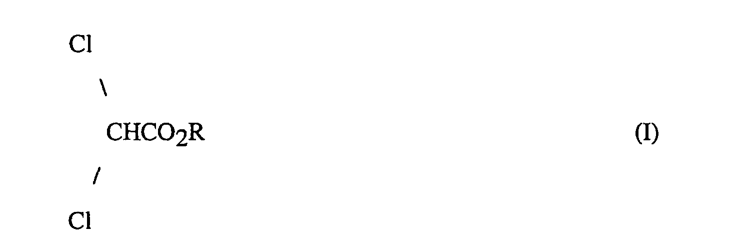 wherein R is selected from hydrogen, (1-10C)alkyl, (3-10C)cycloalkyl, (3-10C)cycloalkyl(1-10C)alkyl and phenyl(1-10C)alkyl in which the phenyl moiety may optionally bear one or more substituents.

6. The use as claimed in claim 5 wherein the agent is administered at a dose in the range 35-50 mg per kg body weight.

7. The use as claimed in claim 5 or 6 wherein the phenyl moiety may optionally bear one or more substituents selected from halogeno, (1-4C)alkyl, (3-6C)alkenyl, (1-4C)alkoxy, cyano, trifluoromethyl, nitro, carboxy, amino, (1-4C)alkylamino, dialkylamino of up to six carbon atoms, (1-4C)alkylthio, (1-4C)alkylsulphinyl, (1-4C)alkylsulphonyl and (1-4C)alkylenedioxy.

8. The use is claimed in 5, 6 or 7 wherein R is selected from (1-10C)alkyl, (3-10C)cycloalkyl, (3-10C)cycloalkyl(1-10C)alkyl and phenyl(1-10C)alkyl in which the phenyl moiety may optionally bear one or more substituents selected from halogeno, (1-4C)alkyl, (3-6C)alkenyl, (1-4C)alkoxy, cyano, trifluoromethyl, nitro, carboxy, amino, (1-4C)alkylamino, dialkylamino of up to six carbon atoms, (1-4C)alkylthio, (1-4C)alkylsulphinyl, (1-4C)alkylsulphonyl and (1-4C)alkylenedioxy.

9. The use as claimed in any one of claims 5, 6, 7 or 8 wherein R is hydrogen.

10. The use of an agent selected from dichloroacetic acid, a salt thereof and an ester of dichloroacetic acid to prepare a medicament which activates pyruvate dehydrogenase in warm blooded animals such as man and is for the treatment of ischaemia in limbs.

11. The use as claimed in claim 10 to prepare a medicament for the treatment of intermittent claudication.

12. The use as claimed in claim 10 or 11 wherein the agent is selected from dichloroacetic acid and salts thereof.

13. The use as claimed in claim 12 wherein the agent is the sodium salt of dichloroacetic acid.

## Patentansprüche

1. Verwendung eines Pyruvatdehydrogenaseaktivierenden Mittels zur Herstellung eines Medikaments, das Pyruvatdehydrogenase in Warmblütern wie dem Menschen aktiviert, zur Behandlung von Ischämie in Gliedmaßen.

2. Verwendung nach Anspruch 1, wobei es sich bei dem Medikament um ein Medikament zur Behandlung von Claudicatio intermittens handelt.

3. Verwendung nach Anspruch 1 oder 2, wobei das Mittel ein Mittel enthält, das PDH-Kinase hemmt.

4. Verwendung nach einem der Ansprüche 1 bis 3, wobei das Mittel Dichloressigsäure, ein Derivat davon oder ein Salz davon enthält.

5. Verwendung nach Anspruch 1, 2, 3 oder 4, wobei das Mittel eine Verbindung der Formel I oder ein pharmazeutisch unbedenkliches Salz davon enthält, 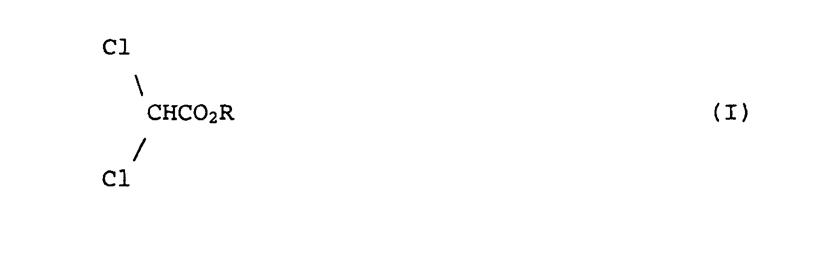 wobei R aus Wasserstoff, (1-10C)Alkyl, (3-10C)Cycloalkyl, (3-10C)Cycloalkyl(1-10C)alkyl und Phenyl(1-10C)alkyl, in welchem die Phenyleinheit gegebenenfalls einen oder mehrere Substituenten tragen kann, ausgewählt ist.

6. Verwendung nach Anspruch 5, wobei man das Mittel in einer Dosis im Bereich von 35-50 mg pro kg Körpergewicht verabreicht.

7. Verwendung nach Anspruch 5 oder 6, wobei die Phenyleinheit gegebenenfalls einen oder mehrere Substituenten ausgewählt aus Halogen, (1-4C)Alkyl, (3-6C)Alkenyl, (1-4C)Alkoxy, Cyano, Trifluormethyl, Nitro, Carboxy, Amino, (1-4C)Alkylamino, Dialkylamin mit bis zu sechs Kohlenstoffatomen, (1-4C)Alkylthio, (1-4C)Alkylsulfinyl, (1-4C)Alkylsulfonyl und (1-4C)Alkylendioxy tragen kann.

8. Verwendung nach Anspruch 5, 6 oder 7, wobei R aus (1-10C)Alkyl, (3-10C)Cycloalkyl, (3-10C)Cycloalkyl(1-10C)alkyl und Phenyl(1-10C)alkyl, in welchem die Phenyleinheit gegebenenfalls einen oder mehrere Substituenten ausgewählt aus Halogen, (1-4C)Alkyl, (3-6C)Alkenyl, (1-4C)Alkoxy, Cyano, Trifluormethyl, Nitro, Carboxy, Amino, (1-4C)Alkylamino, Dialkylamino mit bis zu sechs Kohlenstoffatomen, (1-4C)Alkylthio, (1-4C)Alkylsulfinyl, (1-4C)Alkylsulfonyl und (1-4C)Alkylendioxy tragen kann.

9. Verwendung nach einem der Ansprüche 5, 6,7 oder 8, wobei R für Wasserstoff steht.

10. Verwendung eines Mittels ausgewählt aus Dichloressigsäure, einem Salz davon und einem Ester von Dichloressigsäure, zur Herstellung eines Medikaments, das Pyruvatdehydrogenase in Warmblütern wie dem Menschen aktiviert, zur Behandlung von Ischämie in Gliedmaßen.

11. Verwendung nach Anspruch 10 zur Herstellung eines Medikaments zur Behandlung von Claudicatio intermittens.

12. Verwendung nach Anspruch 10 oder 11, wobei das Mittel aus Dichloressigsäure und Salzen davon ausgewählt ist.

13. Verwendung nach Anspruch 12, wobei es sich bei dem Mittel um das Natriumsalz von Dichloressigsäure handelt.

## Revendications

1. Utilisation d'un agent qui active la pyruvate déshydrogénase pour préparer un médicament qui active la pyruvate déshydrogénase chez les animaux à sang chaud tels que l'homme, pour le traitement de l'ischémie dans les membres.

2. Utilisation selon la revendication 1, dans laquelle le médicament est destiné au traitement de la claudication intermittente.

3. Utilisation selon la revendication 1 ou 2, dans laquelle l'agent contient un agent qui inhibe la PDH-kinase.

4. Utilisation selon l'une quelconque des revendications 1 à 3, dans laquelle l'agent contient de l'acide dichloroacétique, un dérivé de celui-ci, ou un sel de celui-ci.

5. Utilisation selon la revendication 1, 2, 3 ou 4, dans laquelle l'agent contient un composé de formule I, ou un sel pharmaceutiquement acceptable de celui-ci 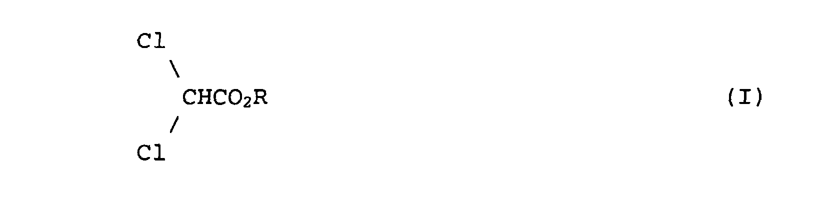 dans laquelle R est sélectionné parmi hydrogène, alkyle à 1-10C, cycloalkyle à 3-10C, (cycloalkyle à 3-10C) (alkyle à 1-10C) et phényl(alkyle à 1-10C) où le groupement phényle peut facultativement porter un ou plusieurs substituants.

6. Utilisation selon la revendication 5, dans laquelle l'agent est administré à une dose dans la gamme de 35 à 50 mg par kg de poids corporel.

7. Utilisation selon la revendication 5 ou 6, dans laquelle le groupement phényle peut facultativement porter un ou plusieurs substituants sélectionnés parmi halogéno, alkyle à 1-4C, alcényle à 3-6C, alcoxy à 1-4C, cyano, trifluorométhyle, nitro, carboxy, amino, alkylamino à 1-4C, dialkylamino contenant jusqu'à six atomes de carbone, alkylthio à 1-4C, alkylsulfinyle à 1-4C, alkylsulfonyle à 1-4C et alkylènedioxy à 1-4C.

8. Utilisation selon la revendication 5, 6 ou 7, dans laquelle R est sélectionné parmi alkyle à 1-10C, cycloalkyle à 3-10C, (cycloalkyle à 3-10C) (alkyle à 1-10C) et phényl(alkyle à 1-10C) où le groupement phényle peut facultativement porter un ou plusieurs substituants sélectionnés parmi halogéno, alkyle à 1-4C, alcényle à 3-6C, alcoxy à 1-4C, cyano, trifluorométhyle, nitro, carboxy, amino, alkylamino à 1-4C, dialkylamino contenant jusqu'à six atomes de carbone, alkylthio à 1-4C, alkylsulfinyle à 1-4C, alkylsulfonyle à 1-4C et alkylènedioxy à 1-4C.

9. Utilisation selon l'une quelconque des revendications 5, 6, 7 ou 8, dans laquelle R est hydrogène.

10. Utilisation d'un agent sélectionné parmi l'acide dichloroacétique, un sel de celui-ci et un ester d'acide dichloroacétique pour préparer un médicament et qui est destiné au traitement de l'ischémie dans les membres.

11. Utilisation selon la revendication 10 pour préparer un médicament pour le traitement de la claudication intermittente.

12. Utilisation selon la revendication 10 ou 11, dans laquelle l'agent est sélectionné parmi l'acide dichloroacétique et des sels de celui-ci.

13. Utilisation selon la revendication 12, dans laquelle l'agent est le sel sodique de l'acide dichloroacétique.
